# EUROPÄISCHE PATENTANMELDUNG

(11) **EP 1 405 604 A1**
(43) Veröffentlichungstag der Anmeldung: **07.04.2004**
(21) Anmeldenummer: 02022198.2
(22) Anmeldetag: 04.10.2002
(51) Int. Cl.: A61B 17/22

(54) **Vorrichtung zur Lokalisation und/oder Diagnose eines Behandlungsbereichs eines menschlichen oder tierischen Körpers**

(71) Anmelder: HMT High Medical Technologies AG, 8574 Lengwil (CH)
(72) Erfinder: Brill, Norbert, 78465 Konstanz (DE); Van Rijn, Dick, 88145 Hergatz (DE); Regenscheit, Stefan, 8274 Gottlieben (CH); Vogler, Jürgen, 78479 Reichenau (DE); Freidinger, Jörg, 78351 Bodman-Ludwigshafen (DE)

(57) **Zusammenfassung**

Eine Vorrichtung zur dualen Lokalisation eines Behandlungsbereichs eines menschlichen oder tierischen Köpers mit einem Röntgensystem, welches auf einer C-bogenförmigen ersten Halterung isozentrisch um eine Achse drehbar angeordnet ist, um den Behandlungsbereich in das Isozentrum zu positionieren und mit einem Ultraschallsystem, bei dem ein Ultraschallkopf an einer um dieselbe Achse drehbaren zweiten Halterung angeordnet ist und bei dem die akustische Achse des Ultraschallkopfes durch das Isozentrum des Röntgensystems verläuft.

## Beschreibung

Die Erfindung betrifft eine Vorrichtung zur Lokalisation und/oder Diagnose eines Behandlungsbereichs eines menschlichen oder tierischen Körpers gemäss dem Oberbegriff des Anspruchs 1.

Lokalisations- und/oder Diagnosesysteme werden in der Medizin beispielsweise zur Ortung von Konkrementen im Körper eines Patienten für die Behandlung mittels akustischer Wellen verwendet.

Aus der US 5 065 761 ist ein Behandlungssystem bekannt mit einer Therapiequelle zur Erzeugung von akustischen Stosswellen, die auf ein zu zerstörendes Konkrement fokussierbar sind, und mit einer Lokalisationsvorrichtung zur Positionserkennung des zu behandelnden Konkrements. Die Lokalisationsvorrichtung besteht aus einem Röntgensystem und einem Ultraschallsystem, die die Lage des zu zerstörenden Konkrements erfassen und damit die Ausrichtung des Konkrements in dem Fokusbereich der Therapiequelle ermöglichen. Bei dieser bekannten Vorrichtung ist das Röntgensystem in einer räumlichen Distanz von der Therapiequelle angeordnet. Der Patient wird auf einer Lagerungsvorrichtung zunächst in das Röntgensystem gebracht, wo das zu behandelnde Konkrement lokalisiert und in eine definierte Position gebracht wird. Anschliessend wird die Lagerungsvorrichtung definiert in eine Behandlungsposition bewegt, so dass das positionierte Konkrement in den Fokus der Therapiequelle gelangt. In der Behandlungsposition wird die Position des zu behandelnden Konkrements mittels des Ultraschallsystems überwacht. Da das Röntgensystem und die Therapiequelle mit dem Ultraschallsystem räumlich voneinander getrennt sind, sind aufwendige Lagerungsund Führungseinrichtungen notwendig, um den in dem Röntgensystem lokalisierten und positionierten Behandlungsbereich definiert in den Fokus der Therapiequelle zu bringen. Die Position des Behandlungsbereichs kann durch das Röntgensystem nur kontrolliert werden, indem die Behandlung unterbrochen wird und der Patient mittels der Lagerungsvorrichtung in das Röntgensystem und anschliessend wieder in die Behandlungsposition zurückbewegt wird.

Aus der DE 199 03 877 C1 ist ein Ultraschallsystem bekannt, welches zur Lokalisation des Behandlungsbereichs direkt an der Therapiequelle befestigt ist. Das Ultraschallsystem ist isozentrisch zu dem Fokus und der akustischen Achse der Therapiequelle bewegbar, um den Behandlungsbereich in dem Fokus der Therapiequelle zu positionieren. Bei dieser Vorrichtung ist das Volumen zur Aufnahme des Körpers des Patienten wegen des geringen Abstandes der Halterung des Ultraschallkopfes von der Therapiequelle beschränkt. Zum Beispiel bei adipösen Patienten kann dies zu Problemen führen. Ein Röntgensystem zur Lokalisation des Behandlungsbereichs ist nicht integriert, so dass eine Röntgenortung räumlich getrennt durchgeführt werden muss.

Der Erfindung liegt die Aufgabe zugrunde, eine Vorrichtung zur Lokalisation und/oder Diagnose eines Behandlungsbereichs zu schaffen, welche eine duale Lokalisation mittels eines Röntgensystems und eines Ultraschallsystems ohne Unterbrechung der Behandlung ermöglicht und ein ausreichendes Arbeitsvolumen zur Aufnahme des Patienten aufweist.

Diese Aufgabe wird erfindungsgemäss gelöst durch eine Vorrichtung mit den Merkmalen des Anspruchs 1.

Vorteilhafte Ausführungen und Weiterbildungen der Erfindung sind in den Unteransprüchen angegeben.

Der wesentliche Gedanke der Erfindung besteht darin, dass das Ultraschallsystem in das Röntgensystem integriert ist und mit diesem auf einen definierten räumlichen Punk ausgerichtet ist. Für eine Anwendung in der Steintherapie kann z. B. das Ultraschallsystem zur Unterstützung der Lokalisation angewendet werden. Beispielsweise kann eine Vorortung unter Anwendung von Ultraschall durchgeführt werden und somit die Röntgen-Strahlenbelastung für Patient und Anwender erheblich reduziert werden.

Zusätzlich ist auch eine Prelokalisation mittels des Röntgensystems und die Real-Time-Überwachung unter Ultraschall möglich. Eine Stein-Positionskontrolle mittels des Röntgensystems kann ohne Unterbrechung der Behandlung erfolgen, da eine Bewegung des Patienten in die Röntgenposition nicht erforderlich ist. Die Behandlungszeit wird damit erheblich reduziert.

Der Aufbau der erfindungsgemässen Vorrichtung ist einfacher und kostensparender zu realisieren.

Ein weiterer Vorteil ergibt sich aus der Möglichkeit der unabhängigen Bewegung des Ultraschallkopfes gegenüber dem Röntgensystem.

Dies wird durch eine entsprechende Freilaufvorrichtung der Ultraschallkopfhalterung gewährleistet.

Gegenüber der bekannten Vorrichtung ergibt sich aufgrund der Integration des Ultraschallsystemes in das Röntgensystem ein grösserer Arbeitsradius des Ultraschallkopfes für den Anwender. Die Behandlung adipöser Patienten wird auch unter Ultraschall möglich.

Gleichzeitig ergibt sich mit einem grösseren Arbeitsvolumen für die Lokalisation unter Ultraschall eine verbesserte Handhabung für den Anwender. Durch den vorhandenen Platz an der Patientenlagerung können die Patientenlagerung unterstützende Vorrichtungen angebracht werden, was wiederum eine effizientere Behandlung gestattet.

Zusätzlich wird eine Bewegung der Ultraschallkopfhalterung und damit des Ultraschallkopfes aus dem Isozentrum des Röntgensystems ermöglicht, die eine Positionierung der Ultraschallkopfhalterung in eine Ruheposition ausserhalb des Patientenlagerungsbereiches gewährleistet.

In einer weiteren Ausführung sind die Halterung des Röntgensystems mit der Halterung des Ultraschallkopfes und mindestens zwei Halteelemente der Halterung des Ultraschallkopfes beweglich mit jeweils einem Verbindungselement ausgeführt.

Die Halterung des Ultraschallkopfes ist derart gestaltet, dass der Ultraschallkopf in einer bestimmten räumlichen Position fixierbar ist und die akustische Achse des Ultraschallkopfes in jeder dieser Positionen durch den Zentralstrahl und insbesondere durch das Isozentrum des Röntgensystems verläuft.

Die Haltekraft der Halterung des Ultraschallkopfes ist einstellbar.

Die Verbindungselemente sind weiterhin so gestaltet, dass sie eine Hemmung der Halteelemente bewirken. Die Halterung des Ultraschallkopfes kann sich somit ohne äussere Krafteinwirkung nicht selbstständig bewegen.

Der Grad der Hemmung lässt sich dabei einstellen. Dies gestattet eine Auslegung der Halterung für verschiedene Ultraschallköpfe.

Die Halterung des Ultraschallkopfes kann sich einerseits ohne äussere Krafteinwirkung nicht selbstständig bewegen und wird andererseits bei geringer äusserer Krafteinwirkung in eine andere Position gebracht. Der Grad der Hemmung kann entsprechend den einschlägigen Normen gewählt werden. Damit können gefährliche Quetschungen und Scherstellen am Patienten vermieden werden. Dies trägt zur Erhöhung des Patientenschutzes bei.

Die Verbindungselemente sowohl zwischen der Halterung des Röntgensystems und dem ersten Halteelement der Halterung des Ultraschallkopfes als auch zwischen dem ersten und zweiten Halteelement der Halterung des Ultraschallkopfes sind sequenziell feststellbar ausgestaltet. Es ist aber auch denkbar, dass sie unabhängig voneinander feststellbar ausgestaltet sind.

Der Feststellmechanismus der Verbindungselemente wird vorzugsweise elektromagnetisch realisiert. Bei Versorgungsunterbruch sind die Verbindungselemente selbsthaltend, sie verlieren nicht ihre selbsthemmende bzw. fixierende Wirkung. Dies gewährleistet eine hohe Patientensicherheit.

Die Halterung für den Ultraschallkopf ist zweckmässigerweise so ausgestaltet, dass Ultraschallköpfe unterschiedlicher Abmessungen adaptierbar sind. Bestehende Systeme können damit in die erfindungsgemässe Vorrichtung integriert werden.

Die erfindungsgemässe Vorrichtung ist für alle Anwendungen in der Human- und Veterinärmedizin geeignet, bei welchen ein zu behandelnder Körperbereich lokalisiert und für die Behandlung positioniert werden muss. Solche Anwendungen sind die Zerstörung von Körperkonkrementen und Tumoren, die Behandlung von Knochenfrakturen und Pseudarthrosen, die orthopädische Behandlung von z. B. Kalkschulter, Epicondylitis usw. und die Rheumatologie und Schmerztherapie. Ebenso ist es möglich, die erfindungsgemässe Vorrichtung für die Biopsie einzusetzen. Hierbei können insbesondere die Halteelemente der Halterung des Ultraschallkopfes mit Positionssensoren ausgestattet sein, die Positionswerte für die Ortsbestimmung des zu behandelnden Bereichs zur Steuerung der Biopsieinstrumente liefern.

Im Folgenden wird die Erfindung anhand von in der Zeichnung dargestellten Ausführungsbeispielen näher erläutert. Es zeigen:
- Figur 1: eine Seitenansicht der erfindungsgemässen Vorrichtung in einer ersten Ausführung
- Figur 2: eine schematische Darstellung der Bewegung des Ultraschalkopfes und
- Figur 3: eine Seitenansicht der erfindungsgemässen Vorrichtung in einer zweiten Ausführung.

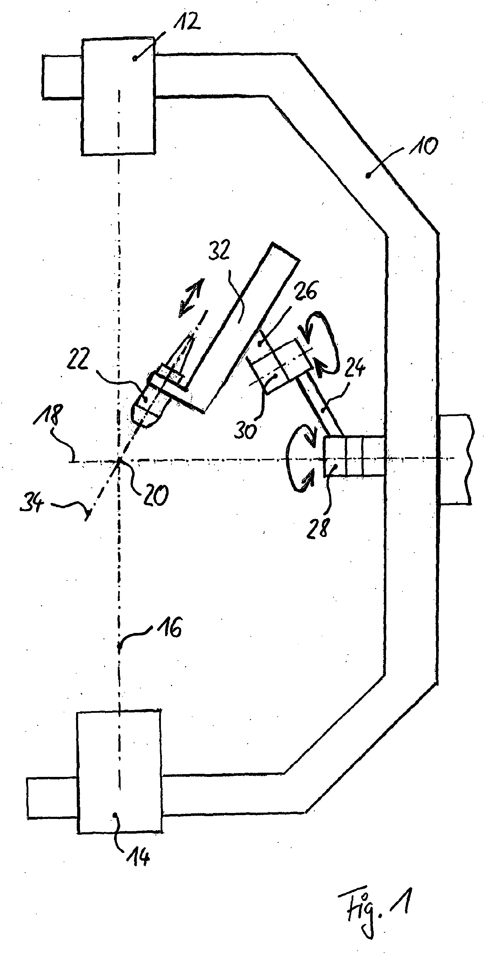

In dem Ausführungsbeispiel der Figur 1 ist eine erste C-bogenförmige Halterung 10 vorgesehen, die an den Enden ihrer Schenkel ein Röntgensystem trägt, welches beispielsweise aus einer Röntgenstrahlquelle 12 und einem Röntgenstrahlempfänger 14 besteht. Der Zentralstrahl des Röntgensystems ist mit 16 bezeichnet. Die C-bogenförmige erste Halterung 10 ist um eine Achse 18 drehbar gelagert. Der Zentralstrahl 16 des Röntgensystems und die Achse 18 schneiden sich in dem Isozentrum 20, welches bei einer Drehung der ersten Halterung 10 um die Achse 18 ortsfest bleibt.

Weiter ist ein Ultraschallkopf 22 vorgesehen, welcher mittels einer zweiten Halterung 24, 26, 28, 30, 32 ebenfalls um die Achse 18 drehbar gelagert ist. Die zweite Halterung 24, 26, 28, 30, 32 ist dabei jedoch unabhängig von der ersten Halterung 10 um die Achse 18 drehbar.

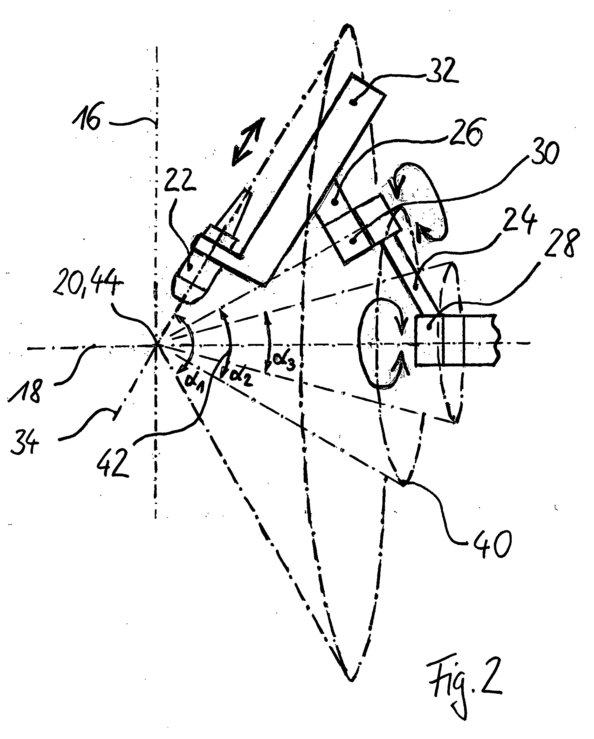

Die zweite Halterung 24, 26, 28, 30, 32 besteht aus einem ersten Halteelement 24 und einem zweiten Halteelement 26, die vorzugsweise als Stab ausgebildet sind. Das erste Halteelement 24 ist mittels eines ersten Verbindungselementes 28 um die Achse 18 schwenkbar gelagert. An dem anderen Ende des ersten Halteelements 24 ist das zweite Halteelement 26 mittels eines zweiten Verbindungselementes 30 schwenkbar gelagert. Das zweite Haltelement 26 weist an seinem freien Ende eine Führung 32 auf, in welcher der Ultraschallkopf 22 in Richtung seiner akustischen Achse 34 verstellbar gehaltert ist. Die Bewegungen des ersten Verbindungselementes 28, des zweiten Verbindungselementes 30 und der Führung 32 sind in den Figuren 1, 2 und 3 jeweils durch Doppelpfeile angedeutet. Durch die zweite Halterung 24, 26, 28, 30, 32 kann der Ultraschallkopf 22, wie in Figur 2 gezeigt, auf einem Kegelmantel 40 bewegt werden, wobei in jeder Position des Ultraschallkopfes 22 auf diesem Kegelmantel 40 die akustische Achse 34 des Ultraschallkopfes 22 den Zentralstrahl 16 des Röntgensystems 12, 14 schneidet. Vorzugsweise liegt dieser Schnittpunkt dabei in dem Isozentrum 20. Der Spitzenwinkel 42 des Kegelmantels 40 ist durch die Ausführung mit den mindestens zwei Verbindungselementen 28, 30 variabel. Die Spitze 44 des Kegelmantels 42 verläuft immer durch das Isozentrum 20.

Die Bewegung des Ultraschallkopfes 22 auf dem Kegelmantel 40 erfolgt durch die Kombination der Führung 32 und der mindestens zwei Verbindungselemente 28, 30. Die Bewegung des Ultraschallkopfes 22 auf einer Kreisbahn um die Achse 18 wird dabei durch das Schwenken des ersten Halteelements 24 um die Achse 18 bewirkt. Der Winkel zwischen der Achse 18 und der akustischen Achse 34 wird durch den Verstellwinkel zwischen dem ersten Halteelement 24 und dem zweiten Halteelement 26 bestimmt. Die Verstellung und Halterung des Ultraschallkopfes 22 in der Führung 32 ermöglicht das Adaptieren unterschiedlicher Ultraschallköpfe 22 und die Anpassung an den Körper des Patienten.

Die Verbindungselemente 28 und 30 und vorzugsweise auch die Führung 32 sind mit einer vorzugsweise einstellbaren Hemmwirkung selbsthaltend. Die Hemmwirkung wird dabei so eingestellt, dass sich die jeweils eingestellte Position des Ultraschallkopfes 22 nicht selbsttätig durch die Schwerkraft oder Erschütterungen verstellt. Andererseits ist die Hemmwirkung so gering, dass der Ultraschallkopf 22 ohne grösseren Widerstand nachgibt, wenn der Körper des Patienten gegen den Ultraschallkopf 22 drückt. Dadurch werden Quetschungen und Verletzungen des Patienten durch den Ultraschallkopf 22 verhindert.

Mittels der Vorrichtung ist eine gleichzeitige oder alternative Lokalisierung und Positionierung des Behandlungsbereichs des Körpers des Patienten in das Isozentrum 20 durch das Röntgensystem 12, 14 und das Ultraschallsystem möglich, ohne dass eine Verlagerung des Patienten erforderlich ist.

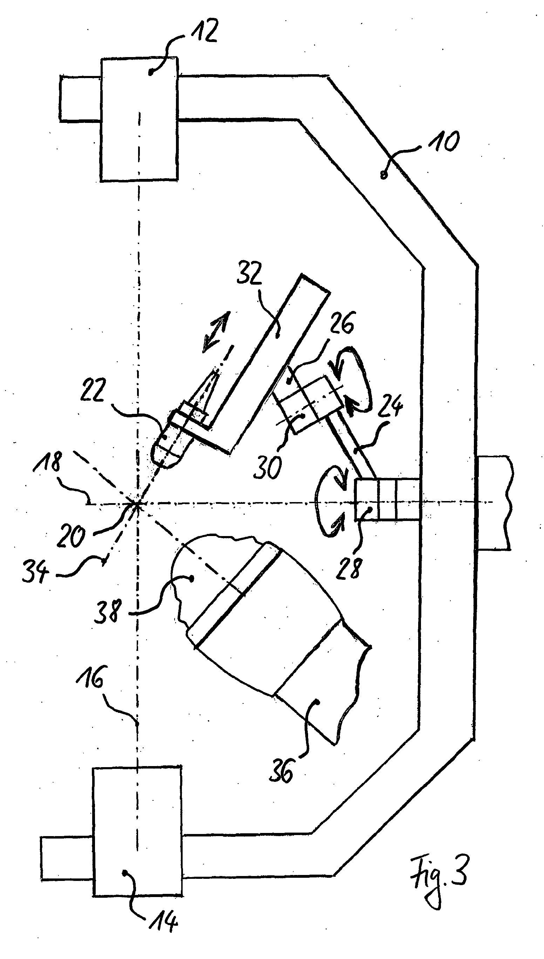

Figur 3 zeigt die Anwendung der Vorrichtung in Verbindung mit einer Therapiequelle 36 zur Erzeugung von fokussierten akustischen Stosswellen.

Die Therapiequelle 36 und die als Lagerachse ausgebildete Achse 18 sind ortsfest in einem gemeinsamen Geräteträger angeordnet, wobei der die Stosswellen erzeugender Therapiekopf 38 der Therapiequelle 36 so angeordnet ist, dass die Stosswellen in das Isozentrum 20 fokussiert werden.

Der zu behandelnde Körperbereich des Patienten wird zunächst mittels des Ultraschallkopfes 22 geortet und in das Isozentrum 20 präpositioniert. Anschliessend kann eine exakte Positionierung des Behandlungsbereiches in das Isozentrum 20 mittels des Röntgensystems 12, 14 durchgeführt werden. Bei der anschliessenden Stosswellen-Behandlung mittels der Therapiequelle 36 kann eine Positionskontrolle mittels des Ultraschallkopfes 22 durchgeführt werden.
Kontrolluntersuchungen über das Behandlungsergebnis, z. B. über die Zerstörung von Konkrementen, können während der Behandlung mittels des Röntgensystems 12, 14 durchgeführt werden. Dabei ist insbesondere vorteilhaft, dass die Lokalisation und Kontrolle mittels des Röntgensystems 12, 14 nur kurzzeitig erforderlich sind, woraus eine geringe Strahlenbelastung des Patienten und des Personals resultiert.

Der Wechsel zwischen Röntgensystem 12, 14 und dem Ultraschallkopf 22 kann jederzeit erfolgen, ohne dass dabei eine Verlagerung des Patienten oder eine Verstellung der Systeme notwendig ist. Die Anordnung des Ultraschallkopfes 22 mittels der zweiten Halterung 24, 26, 28, 30, 32 an der Achse 18 ergibt einen grossen Freiraum des Ultraschallkopfes 22 für die Aufnahme und Lagerung des Patienten.

### Bezugszeichenliste

- 10: erste Halterung
- 12: Röntgenstrahlquelle
- 14: Röntgenstrahlempfänger
- 16: Zentralstrahl
- 18: Achse
- 20: Isozentrum
- 22: Ultraschallkopf

- 24: erstes Halteelement
- 26: zweites Halteelement
- 28: erstes Verbindungselement
- 30: zweites Verbindungselement
- 32: Führung
- 34: akustische Achse
- 36: Therapiequelle
- 38: Therapiekopf
- 40: Kegelmantel
- 42: Spitzenwinkel des Kegelmantels
- 44: Spitze des Kegelmantels

## Patentansprüche

1. Vorrichtung zur Lokalisation und/oder Diagnose eines Behandlungsbereichs eines menschlichen oder tierischen Köpers, mit einem Röntgensystem (12, 14), welches auf einer C-bogenförmigen ersten Halterung (10) isozentrisch um eine Achse (18) drehbar angeordnet ist, um den Behandlungsbereich in das Isozentrum (20) zu positionieren, und mit einem Ultraschallsystem,
**dadurch gekennzeichnet, dass** ein Ultraschallkopf (22) an einer um dieselbe Achse (18) drehbaren zweiten Halterung (24, 26, 28, 30, 32) angeordnet ist und dass die akustische Achse (34) des Ultraschallkopfes (22) durch den Zentralstrahl (16) und insbesondere durch das Isozentrum (20) des Röntgensystems (12, 14) verläuft.

2. Vorrichtung nach Anspruch 1,
**dadurch gekennzeichnet, dass** die Achsen (18) der ersten Halterung (10) und zweiten Halterung (24, 26, 28, 30, 32) in einer gemeinsamen Lagerachse fluchten.

3. Vorrichtung nach Anspruch 1,
**dadurch gekennzeichnet, dass** die zweite Halterung (24, 26, 28, 30, 32) mit mindestens zwei Halteelementen (24, 26) ausgebildet ist.

4. Vorrichtung nach Anspruch 3,
**dadurch gekennzeichnet, dass** die mindestens zwei Halteelemente (24, 26) stabförmig ausgebildet sind.

5. Vorrichtung nach Anspruch 3,
**dadurch gekennzeichnet, dass** ein erstes Halteelement (24) um die Achse (18) der C-bogenförmigen Halterung (10) des Röntgensystems (12, 14) drehbar ist.

6. Vorrichtung nach Anspruch 5,
**dadurch gekennzeichnet, dass** die Drehung des Halteelements (24) unabhängig von der Drehung der C-bogenförmigen Halterung (10) des Röntgensystems (12, 14) erfolgt.

7. Vorrichtung nach Anspruch 3,
**dadurch gekennzeichnet, dass** die erste Halterung (10) mit der zweiten Halterung (24, 26, 28, 30, 32) und die mindestens zwei Haltelemente (24, 26) mit jeweils einem Verbindungselement (28, 30) gelenkig verbunden sind.

8. Vorrichtung nach Anspruch 7,
**dadurch gekennzeichnet, dass** die Verbindungselemente (28, 30) derart gestaltet sind, dass der Ultraschallkopf (22) auf eine bestimmte räumliche Position fixierbar ist.

9. Vorrichtung nach Anspruch 8,
**dadurch gekennzeichnet, dass** das erste Verbindungselement (28) derart gestaltet ist, dass die akustische Achse (34) des Ultraschallkopfes (22) aus dem Zentralstrahl (16) des Röntgensystems (12, 14) bewegbar ist.

10. Vorrichtung nach Anspruch 8,
**dadurch gekennzeichnet, dass** die Haltekraft der Verbindungselemente (28, 30) zur Fixierung der zweiten Halterung (24, 26, 28, 30, 32) einstellbar ist.

11. Vorrichtung nach Anspruch 7,
**dadurch gekennzeichnet, dass** die Verbindungselemente (28, 30) selbsthemmend ausgeführt sind.

12. Vorrichtung nach Anspruch 11,
**dadurch gekennzeichnet, dass** die selbsthemmende Haltekraft einstellbar ist.

13. Vorrichtung nach Anspruch 7,
**dadurch gekennzeichnet, dass** die Halteelemente (24, 26) über die Verbindungselemente (28, 30) sequenziell feststellbar sind.

14. Vorrichtung nach Anspruch 7,
**dadurch gekennzeichnet, dass** die Haltelemente (24, 26) über die Verbindungselemente (28, 30) unabhängig voneinander feststellbar sind.

15. Vorrichtung nach Anspruch 7,
**dadurch gekennzeichnet, dass** jedes Halteelement (24, 26) mit einem Positionssensor ausgestattet ist.

16. Vorrichtung nach Anspruch 7,
**dadurch gekennzeichnet, dass** der Ultraschallkopf (22) in einer Führung (32) gelagert ist.

17. Vorrichtung nach Anspruch 16,
**dadurch gekennzeichnet, dass** die Führung (32) für Ultraschallköpfe (22) unterschiedlicher Abmessungen ausgeführt ist.

18. Vorrichtung nach Anspruch 1,
**dadurch gekennzeichnet, dass** die Vorrichtung ortsfest in Bezug auf eine Stosswellen-Therapiequelle (36, 38) angeordnet ist.

19. Vorrichtung nach Anspruch 18,
**dadurch gekennzeichnet, dass** das Isozentrum (20) des Röntgensystems (12, 14) mit dem Fokus der von der Therapiequelle (36, 38) erzeugten Stosswellen zusammenfällt.

20. Vorrichtung nach den Ansprüchen 18 und 19,
**dadurch gekennzeichnet, dass** die Vorrichtung und die Therpiequelle (36) mechanisch fest in einem gemeinsamen Geräteträger angeordnet sind.

21. Vorrichtung nach den Ansprüchen 18 und 19,
**dadurch gekennzeichnet, dass** die Vorrichtung und die Therpiequelle (36) mechanisch koppelbar und justierbar ausgeführt sind.

22. Vorrichtung nach Anspruch 1,
**dadurch gekennzeichnet,dass** die zweite Halterung (24, 26, 28, 30, 32) an die C-bogenförmige Halterung (10) adaptierbar ist.
